# EUROPÄISCHE PATENTSCHRIFT

(11) **EP 3 464 427 B1**
(45) Veröffentlichungstag und Bekanntmachung des Hinweises auf die Patenterteilung: **06.01.2021**
(21) Anmeldenummer: 17726895.0
(22) Anmeldetag: 23.05.2017
(51) Int. Cl.: C08J 3/24, C08F 220/06, B01J 20/30, B01J 20/26, A61L 15/60

(54) **VERFAHREN ZUR HERSTELLUNG VON SUPERABSORBERN**
METHOD FOR THE PRODUCTION OF SUPERABSORBERS
PROCÉDÉ DE FABRICATION DE SUPERABSORBANTS

(30) Priorität: 31.05.2016 EP 16172267
(43) Veröffentlichungstag der Anmeldung: 10.04.2019
(73) Patentinhaber: BASF SE, 67056 Ludwigshafen am Rhein (DE)
(72) Erfinder: GRÜNEWALD, Gerald, 67056 Ludwigshafen (DE); FUNK, Ruediger, 67056 Ludwigshafen (DE); WEISMANTEL, Matthias, 67056 Ludwigshafen (DE)
(74) Vertreter: BASF IP Association
(86) Internationale Anmeldenummer: PCT/EP2017/062327
(87) Internationale Veröffentlichungsnummer: WO 2017/207330

(56) Entgegenhaltungen:
- EP-A1- 2 253 375
- EP-A1- 2 338 918
- EP-A1- 2 415 822
- WO-A1-2012/152647
- US-A1- 2010 249 320

## Beschreibung

Die vorliegende Erfindung betrifft ein Verfahren zur Herstellung von Superabsorbern, umfassend Polymerisation, Trocknung, Brechen, pneumatischer Förderung, Zerkleinerung und Klassierung, wobei die Gastemperatur am Ende der pneumatischen Förderung von 50 bis 95°C beträgt.

Superabsorber werden zur Herstellung von Windeln, Tampons, Damenbinden und anderen Hygieneartikeln, aber auch als wasserzurückhaltende Mittel im landwirtschaftlichen Gartenbau verwendet. Die Superabsorber werden auch als wasserabsorbierende Polymere bezeichnet.

Die Herstellung von Superabsorbern wird in der Monographie "Modern Superabsorbent Polymer Technology", F.L. Buchholz und A.T. Graham, Wiley-VCH, 1998, Seiten 71 bis 103, beschrieben.

Die Eigenschaften der Superabsorber können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm² (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49,2 g/cm² (AUL0.7psi), werden Superabsorberpartikel im allgemeinen oberflächennachvernetzt. Dadurch steigt der Vernetzungsgrad der Partikeloberfläche, wodurch die Absorption unter einem Druck von 49,2 g/cm² (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der Polymerpartikel kovalente Bindungen bilden können.

EP 1 118 633 A2, EP 1 130 045 A2, EP 2 258 749 A1 und WO 2012/119969 A1 und EP 2 415 822 offenbaren Verfahren zur Herstellung von Superabsorbern.

EP 1 118 633 A2 lehrt die Beheizung von Oberflächen bei Lagerung und Transport von Superabsorbern.

EP 1 130 045 A2 lehrt die Kühlung zwischen der Trocknung wässriger Polymergele und der anschließenden Zerkleinerung.

EP 2 258 749 A1 lehrt die Verwendung trockener Gase und glatter Rohrleitungen bei der pneumatischen Förderung.

WO 2012/119969 A1 lehrt die pneumatische Förderung zwischen Zerkleinerung und Klassierung.

Aufgabe der vorliegenden Erfindung war die Bereitstellung eines verbesserten Verfahrens zur Herstellung von Superabsorbern, wobei insbesondere Störungen beim Klassieren des Grundpolymers vermindert werden sollen.

Gelöst wurde die Aufgabe durch ein Verfahren zur Herstellung von Superabsorbern durch Polymerisation einer Monomerlösung oder-suspension, enthaltend
a) ein ethylenisch ungesättigtes, säuregruppentragendes Monomer, das zumindest teilweise neutralisiert ist,
b) mindestens einen Vernetzer,
c) mindestens einen Initiator,
d) optional ein mit den unter a) genannten Monomeren copolymerisierbares ethylenisch ungesättigtes Monomer und
e) optional ein oder mehrere wasserlösliche Polymere,
umfassend die Schritte
i) Polymerisation der Monomerlösung oder -suspension,
ii) optional Zerkleinerung des in Schritt i) erhaltenen Polymergels,
iii) Trocknung des in Schritt i) oder Schritt ii) erhaltenen Polymergels in einem Umluftbandtrockner mit mehreren Heizzonen und mindestens einer Kühlzone,
iv) Brechen des in Schritt iii) erhaltenen getrockneten Polymergels,
v) optional Grobzerkleinung der in Schritt iv) erhaltenen Polymerpartikel,
vi) pneumatischer Förderung der in Schritt iv) oder Schritt v) erhaltenen Polymerpartikel,
vii) optional Abtrennung unvollständig getrockneter Polymerpartikel aus den in Schritt vi) erhaltenen Polymerpartikeln, wobei die verbleibenden getrockneten Polymerpartikel in Schritt viii), Schritt ix) oder Schritt x) weiterverarbeitet werden,
viii) optional Klassierung der in Schritt vi) oder Schritt vii) erhaltenen Polymerpartikel, wobei die Grobfraktion dem Schritt ix) oder Schritt x) zugeführt wird,
ix) optional Zwischenlagerung der in Schritt vi), Schritt vii) oder Schritt viii) erhaltenen Polymerpartikel,
x) Zerkleinerung der Polymerpartikel der in Schritt vi), Schritt vii), Schritt viii) oder Schritt ix) erhaltenen Polymerpartikel,
xi) optional pneumatischer Förderung der in Schritt x) erhaltenen Polymerpartikel,
xii) Klassierung der in Schritt x) oder Schritt xi) erhaltenen Polymerpartikel und
xiii) optional Oberflächennachvernetzung der in Schritt viii) und/oder Schritt xii) erhaltenen klassierten Polymerpartikel,
dadurch gekennzeichnet, dass die Gastemperatur am Ende der pneumatischen Förderung in Schritt vi) von 50 bis 95°C, vorzugsweise von 53 bis 90°C, besonders bevorzugt von 56 bis 85°C, ganz besonders bevorzugt von 59 bis 80°C, beträgt.

Die pneumatische Förderung wird beispielsweise in WO 2007/104657 A2,
WO 2007/104673 A2, WO 2007/104676 A1, EP 2 471 847 A1 und EP 2 471 848 A1 beschrieben.

In einer bevorzugten Ausführungsform der vorliegenden Erfindung werden die in Schritt vi) erhaltenen Polymerpartikel oder die in oder Schritt vii) nach der Abtrennung der unvollständig getrockneter Polymerpartikel verbleibenden Polymerpartikel in Schritt viii) klassiert, wobei die Grobfraktion Schritt ix) oder Schritt x) zugeführt wird.

Die Verweilzeit zwischen dem Ende der Trocknung in Schritt iii) und dem Ende der pneumatischen Förderung in Schritt vi) beträgt vorzugsweise weniger als 30 Minuten, besonders bevorzugt weniger als 20 Minuten, ganz besonders bevorzugt weniger als 10 Minuten.

Die Verweilzeit in der pneumatischen Förderung kann als Quotient aus Länge der Förderleitung in m und der mittleren Gasgeschwindigkeit in m/s ermittelt werden, wobei die mittlere Gasgeschwindigkeit das arithmetrische Mittel aus Gasanfangsgeschwindigkeit und Gasendgeschwindigkeit ist. Die Verweilzeit in etwaigen Zwischenbehältern zwischen dem Ende der Trocknung in Schritt iii) und der pneumatischen Förderung in Schritt vi) ist zu addieren. Genauer kann die Verweilzeit über Markierungsexperimente, z.B. eingefärbte Polymerpartikel, bestimmt werden.

In weiteren bevorzugten Ausführungsformen der vorliegenden Erfindung betragen die Gastemperaturen am Ende der pneumatischen Förderung in Schritt vi) und die Verweilzeiten zwischen dem Ende der Trocknung in Schritt iii) und dem Ende der pneumatischen Förderung in Schritt vi) beispielsweise von 50 bis 95°C und weniger als 30 Minuten bzw. von 50 bis 95°C und weniger als 20 Minuten bzw. von 50 bis 95°C und weniger als 10 Minuten bzw. von 53 bis 90°C und weniger als 30 Minuten bzw. von 53 bis 90°C und weniger als 20 Minuten bzw. von 53 bis 90°C und weniger als 10 Minuten bzw. von 56 bis 85°C und weniger als 30 Minuten bzw. von 56 bis 85°C und weniger als 20 Minuten bzw. von 56 bis 85°C und weniger als 10 Minuten bzw. von 59 bis 80°C und weniger als 30 Minuten bzw. von 59 bis 80°C und weniger als 20 Minuten bzw. von 59 bis 80°C und weniger als 10 Minuten.

Der Feuchtegehalt der Polymerpartikel in Schritt x) beträgt vorzugsweise von 0,5 bis 10 Gew.-%, besonders bevorzugt von 1 bis 6 Gew.-%, ganz besonders bevorzugt von 1,5 bis 4 Gew.-%, wobei der Feuchtegehalt gemäß der von der EDANA empfohlenen Testmethode Nr. WSP 230.2-05 "Mass Loss Upon Heating" ermittelt wird.

In weiteren bevorzugten Ausführungsformen der vorliegenden Erfindung betragen die Gastemperaturen am Ende der pneumatischen Förderung in Schritt vi), die Verweilzeiten zwischen dem Ende der Trocknung in Schritt iii) und dem Ende der pneumatischen Förderung in Schritt vi) und der Feuchtegehalt der Polymerpartikel in Schritt x) beispielsweise von 50 bis 95°C, weniger als 30 Minuten und von 0,5 bis 10 Gew.-% bzw. von 50 bis 95°C, weniger als 30 Minuten und von 1 bis 6 Gew.-% bzw. von 50 bis 95°C, weniger als 30 Minuten und von 1,5 bis 4 Gew.-% bzw. von 50 bis 95°C, weniger als 20 Minuten und von 0,5 bis 10 Gew.-% bzw. von 50 bis 95°C, weniger als 20 Minuten und von 1 bis 6 Gew.-% bzw. von 50 bis 95°C, weniger als 20 Minuten und von 1,5 bis 4 Gew.-% bzw. von 50 bis 95°C, weniger als 10 Minuten und von 0,5 bis 10 Gew.-% bzw. von 50 bis 95°C, weniger als 10 Minuten und von 1 bis 6 Gew.-% bzw. von 50 bis 95°C, weniger als 10 Minuten und von 1,5 bis 4 Gew.-% bzw. von 53 bis 90°C, weniger als 30 Minuten und von 0,5 bis 10 Gew.-% bzw. von 53 bis 90°C, weniger als 30 Minuten und von 1 bis 6 Gew.-% bzw. von 53 bis 90°C, weniger als 30 Minuten und von 1,5 bis 4 Gew.-% bzw. von 53 bis 90°C, weniger als 20 Minuten und von 0,5 bis 10 Gew.-% bzw. von 53 bis 90°C, weniger als 20 Minuten und von 1 bis 6 Gew.-% bzw. von 53 bis 90°C, weniger als 20 Minuten und von 1,5 bis 4 Gew.-% bzw. von 53 bis 90°C, weniger als 10 Minuten und von 0,5 bis 10 Gew.-% bzw. von 53 bis 90°C, weniger als 10 Minuten und von 1 bis 6 Gew.-% bzw. von 53 bis 90°C, weniger als 10 Minuten und von 1,5 bis 4 Gew.-% bzw. von 56 bis 85°C, weniger als 30 Minuten und von 0,5 bis 10 Gew.-% bzw. von 56 bis 85°C, weniger als 30 Minuten und von 1 bis 6 Gew.-% bzw. von 56 bis 85°C, weniger als 30 Minuten und von 1,5 bis 4 Gew.-% bzw. von 56 bis 85°C, weniger als 20 Minuten und von 0,5 bis 10 Gew.-% bzw. von 56 bis 85°C, weniger als 20 Minuten und von 1 bis 6 Gew.-% bzw. von 56 bis 85°C, weniger als 20 Minuten und von 1,5 bis 4 Gew.-% bzw. von 56 bis 85°C, weniger als 10 Minuten und von 0,5 bis 10 Gew.-% bzw. von 56 bis 85°C, weniger als 10 Minuten und von 1 bis 6 Gew.-% bzw. von 56 bis 85°C, weniger als 10 Minuten und von 1,5 bis 4 Gew.-% bzw. von 59 bis 80°C, weniger als 30 Minuten und von 0,5 bis 10 Gew.-% bzw. von 59 bis 80°C, weniger als 30 Minuten und von 1 bis 6 Gew.-% bzw. von 59 bis 80°C, weniger als 30 Minuten und von 1,5 bis 4 Gew.-% bzw. von 59 bis 80°C, weniger als 20 Minuten und von 0,5 bis 10 Gew.-% bzw. von 59 bis 80°C, weniger als 20 Minuten und von 1 bis 6 Gew.-% bzw. von 59 bis 80°C, weniger als 20 Minuten und von 1,5 bis 4 Gew.-% bzw. von 59 bis 80°C, weniger als 10 Minuten und von 0,5 bis 10 Gew.-% bzw. von 59 bis 80°C, weniger als 10 Minuten und von 1 bis 6 Gew.-% bzw. von 59 bis 80°C, weniger als 10 Minuten und von 1,5 bis 4 Gew.-%.

In einer besonders bevorzugten Ausführungsform der vorliegenden Erfindung wird die Kühlleistung der mindestens einen Kühlzone in Schritt iii) zur Regelung der Gastemperatur am Ende der pneumatischen Förderung in Schritt vi) verwendet.

Der vorliegenden Erfindung liegt die Erkenntnis zugrunde, dass die Gastemperatur am Ende der pneumatischen Förderung einen entscheidenden Einfluss auf den störungsfreien Betrieb der anschließenden Klassierung hat. Diese Temperatur lässt sich leicht über die Kühlleistung am Ende des zur Trocknung des Polymergels verwendeten Umluftbandtrockners einstellen. Das getrocknete Polymergel wird in Schritt iv) vorzugsweise mittels einer Stachelwalze oder eines Kreuzflügelzerkleinerers gebrochen. Ein Kreuzflügelzerkleinerer umfasst eine Welle, an der eine Vielzahl von Barren aufgenommen ist. Neben den auf der Welle angeordneten Barren umfasst der Kreuzflügelzerkleinerer eine Vielzahl fest montierter Barren, die in Zwischenräume der auf der Welle angeordneten Barren eingreifen. Das in den Kreuzflügelzerkleinerer eingegebene getrocknete Polymergel fällt auf die fest montierten Barren und bleibt auf diesen liegen. Durch die sich mit der Welle mitdrehenden Barren wird das getrocknete Polymergel gebrochen.

Die Polymerpartikel werden in Schritt x) vorzugsweise mittels eines mehrstufigen Walzenstuhls zerkleinert. Geeignete Walzenstühle werden beispielsweise in der Monographie "Modern Superabsorbent Polymer Technology", F.L. Buchholz und A.T. Graham, Wiley-VCH, 1998, Seiten 93 bis 95, beschrieben.

Die Polymerpartikel werden in Schritt viii) und/oder Schritt xii) vorzugsweise mittels einer Taumelsiebmaschine klassiert. Geeignete Taumelsiebmaschinen werden beispielsweise in EP 0 855 232 A2 und WO 2006/0574816 A1 beschrieben.

Die in Schritt iv) erhaltenen Polymerpartikel werden vorzugsweise in Schritt v) mittels eines Walzenbrechers grobzerkleinert. Ein Walzenbrecher besteht aus zwei gegenläufig rotierenden, ggf. mit Zähnen oder Zapfen bestückten, Walzen zwischen denen die Polymerpartikel zerbrochen werden können. Die Walzen des Walzenbrechers weisen eine im Wesentlichen glatte Oberfläche auf, so dass die Polymerpartikel nicht gemahlen oder zerrieben werden.

Die in Schritt x) erhaltenen Polymerpartikel können anschließend in Schritt xi) pneumatisch gefördert werden.

Aus den in Schritt vi) erhaltenen Polymerpartikeln werden vorzugsweise in Schritt vii) unvollständig getrocknete Polymerpartikel abgetrennt. Die Abtrennung unvollständig getrockneter Polymerpartikel wird beispielsweise in EP 0 948 997 A2 und WO 207/057350 A1 beschrieben.

Die in Schritt vii) erhaltenen Polymerpartikel können in Schritt viii) klassiert werden.

Die in Schritt vi), Schritt vii) oder Schritt viii) erhaltenen Polymerpartikel können anschließend in Schritt ix) zwischengelagert werden. Die hierzu geeigneten Behälter oder Silos unterliegen keiner Beschränkung.

Die in Schritt viii) und/oder Schritt xii) erhaltenen Polymerpartikel werden vorzugsweise in Schritt xiii) oberflächennachvernetzt.

Im Folgenden wird die Herstellung der Superabsorber näher erläutert:
Die Superabsorber werden in Schritt i) durch Polymerisation einer Monomerlösung oder-suspension hergestellt und sind üblicherweise wasserunlöslich.
Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.
Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.
Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 02/055469 A1, der WO 03/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomers a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 03/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,25 bis 1,5 Gew.-%, besonders bevorzugt 0,3 bis 1,2 Gew.-%, ganz besonders bevorzugt 0,4 bis 0,8 Gew.-%, jeweils bezogen auf unneutralisiertes Monomer a). Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit die Löslichkeit überschreitendem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren für die Polymerisation in Schritt i) sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Schritt ii) zerkleinert werden muss, beispielsweise in einem Extruder oder Kneter.

Zur Verbesserung der Trocknungseigenschaften kann das mittels eines Kneters erhaltene zerkleinerte Polymergel zusätzlich in Schritt ii) extrudiert werden.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Das Polymergel wird dann in Schritt iii) mit einem Umluftbandtrockner getrocknet bis der Restfeuchtegehalt vorzugsweise 0,5 bis 10 Gew.-%, besonders bevorzugt 1 bis 6 Gew.-%, ganz besonders bevorzugt 1,5 bis 4 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Polymergels beträgt vor der Trocknung vorzugsweise von 25 und 90 Gew.-%, besonders bevorzugt von 35 bis 70 Gew.-%, ganz besonders bevorzugt von 40 bis 60 Gew.-%. Anschließend wird das getrocknete Polymergel in Schritt iv) gebrochen und optional in Schritt v) grob zerkleinert.

Das getrocknete Polymergel wird hiernach in Schritt vi) pneumatisch gefördert, in Schritt x) zerkleinert und in Schritt xii) klassiert, wobei zur Zerkleinerung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion Schritt xii) abgetrennten Polymerpartikel beträgt vorzugsweise von 150 bis 850 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Polymerpartikeln mit einer Partikelgröße von größer 150 µm beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt, vorzugsweise vor, während oder unmittelbar nach der Polymerisation in Schritt i), d.h. vor der Trocknung des Polymergels in Schritt iii). Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung in Schritt xiii) oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation in Schritt i) zugesetzt. Es ist aber auch möglich die zu kleinen Polymerpartikel in einem dem Polymerisationsreaktor nachgeschalteten Schritt ii), beispielsweise in einem Kneter oder Extruder, in das Polymergel einzuarbeiten.

Werden die zu kleinen Polymerpartikel sehr früh zugesetzt, beispielsweise bereits zur Monomerlösung, so wird dadurch die Zentrifugenretentionskapazität (CRC) der erhaltenen Polymerpartikel gesenkt. Dies kann aber beispielsweise durch Anpassung der Einsatzmenge an Vernetzer b) kompensiert werden.

Der Anteil an Polymerpartikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Der Anteil an Polymerpartikeln mit einer Partikelgröße von höchstens 600 µm, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Quellgeschwindigkeit. Daher sollte der Anteil zu großer Polymerpartikel ebenfalls niedrig sein.

Zu große Polymerpartikel werden daher üblicherweise abgetrennt und in die Zerkleinerung in Schritt x) rückgeführt. Werden die Polymerpartikel vor der Zerkleinerung in Schritt x) in Schritt ix) zwischengelagert, so werden die abgetrennten zu großen Polymerpartikel vorzugsweise in die Zwischenlagerung in Schritt ix) rückgeführt.

Die Polymerpartikel können zur weiteren Verbesserung der Eigenschaften in Schritt xiii) oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 03/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben
Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,01 bis 0,8 Gew.-%, jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächennachvernetzt.

Bevorzugte Reaktionstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die Polymerpartikel nach der Oberflächennachvernetzung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die Polymerpartikel auf vorzugsweise 40 bis 90°C, besonders bevorzugt 45 bis 80°C, ganz besonders bevorzugt 50 bis 70°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 40 bis 120°C, besonders bevorzugt bei 50 bis 110°C, ganz besonders bevorzugt bei 60 bis 100°C, durchgeführt. Bei zu niedrigen Temperaturen neigen die Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Oberflächennachvernetzung durchgeführt. Geeignete Beschichtungen zur Verbesserung der Quellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

### Beispiele

### Beispiel 1

Durch kontinuierliches Mischen von entionisiertem Wasser, 50 gew.-%iger Natronlauge und Acrylsäure wurde eine Acrylsäure/Natriumacrylatlösung hergestellt, so dass der Neutralisationsgrad 71,3 mol-% entsprach. Der Feststoffgehalt der Monomerlösung betrug 38,8 Gew.-%.

Als mehrfach ethylenisch ungesättigter Vernetzer wurde Polyethylenglykol-400-diacrylat (Diacrylat ausgehend von einem Polyethylenglykol mit einem mittleren Molgewicht von 400 g/mol) verwendet. Die Einsatzmenge betrug 2 kg Vernetzer pro t Monomerlösung.

Zur Initiierung der radikalischen Polymerisation wurden pro t Monomerlösung 1,03 kg einer 0,25gew.-%igen wässriger Wasserstoffperoxidlösung, 3,10 kg einer 15gew.-%igen wässrigen Natriumperoxodisulfatlösung und 1,05 kg einer 1gew.-%igen wässrigen Ascorbinsäurelösung eingesetzt.

Der Durchsatz der Monomerlösung betrug 20 t/h. Die Reaktionslösung hatte am Zulauf eine Temperatur von 23,5°C.

Die einzelnen Komponenten wurden in folgenden Mengen kontinuierlich in einen Reaktor vom Typ List Contikneter mit einem Volumen 6,3m³ (LIST AG, Arisdorf, Schweiz) dosiert:

| | |
|---|---|
| 20 t/h | Monomerlösung |
| 40 kg/h | Polyethylenglykol-400-diacrylat |
| 82,6 kg/h | Wasserstoffperoxidlösung/Natriumperoxodisulfatlösung |
| 21 kg/h | Ascorbinsäurelösung |

Zwischen dem Zugabepunkt für den Vernetzer und den Zugabestellen für die Initiatoren wurde die Monomerlösung mit Stickstoff inertisiert.

Nach ca. 50% der Verweilzeit wurden im Verfahren anfallende Polymerpartikel mit einer Partikelgröße von weniger als 150 µm (1000 kg/h) in den Reaktor dosiert. Die Verweilzeit der Reaktionsmischung im Reaktor betrug 15 Minuten.

Das erhaltene Polymergel wurde auf einen Umluftbandtrockner aufgegeben. Der Umluftbandtrockner hatte sechs Heizzonen und eine Kühlzone. Auf dem Umluftbandtrockner wurde das Polymergel kontinuierlich mit einem Luft/Gasgemisch umströmt und getrocknet. Die Verweilzeit im Bandtrockner betrug 37 Minuten. In der Kühlzone des Umluftbandtrockners wurde das Polymergel auf 100°C abgekühlt.

Das getrocknete Polymergel wurde mittels eines Kreuzflügelzerkleinerers gebrochen und mittels eines Walzenbrechers grobzerkleinert. Anschließend wurden die Polymerpartikel pneumatisch gefördert (pneumatische Förderung 1) und die unvollständig getrockneten Polymerpartikel abgetrennt. Die Gastemperatur am Ende der pneumatischen Förderung 1 betrug 75°C. Die Verweilzeit zwischen dem Ende der Trocknung und dem Ende der pneumatischen Förderung 1 betrug ca. 2 Minuten.

Zur Abtrennung der unvollständig getrockneten Polymerpartikel wurde mittels einer Vibrationssiebmaschine klassiert. Es wurden Siebe mit einer Maschenweise von 8 mm und 12 mm eingesetzt. Die Polymerpartikel mit einer Partikelgröße von weniger als 8 mm wurden in einem Silo zwischengelagert.

Anschließend wurden die Polymerpartikel mittels eines zweistufigen Walzenstuhls zerkleinert, pneumatisch gefördert (pneumatische Förderung 2) und mittels einer Taumelsiebmaschine klassiert. Der Wassergehalt der Polymerpartikel betrug 2,5 Gew.-%.

Polymerpartikel mit einer Partikelgröße von weniger als 150 µm wurden in den Reaktor zurückgeführt. Polymerpartikel mit einer Partikelgröße von größer 850 µm wurden in das Silo zurückgeführt. Polymerpartikel mit einer Partikelgröße im Bereich von 150 bis 850 µm wurden oberflächennachvernetzt. Die Klassierung lief mehrere Wochen störungsfrei.

Die Polymerpartikel wurden in einem Schugi Flexomix® (Hosokawa Micron B.V., Doetinchem, Niederlande mit einer Oberflächennachvernetzerlösung beschichtet und anschließend in einem NARA Paddle Dryer (GMF Gouda, Waddinxveen, Niederlande) 45 Minuten bei 190°C getrocknet.

Es wurden folgende Mengen in den Schugi Flexomix® dosiert:

| | |
|---|---|
| 7,5 t/h | Polymerpartikel |
| 270,0 kg/h | Oberflächennachvernetzerlösung |

Die Oberflächennachvernetzerlösung enthielt 2,8 Gew.-% 2-Hydroxyethyl-2 oxazolidon, 2,8 Gew.-% Aluminiumsulfat, 66,1 Gew.-% entionisiertes Wasser und 28,3 Gew.-% Isopropanol.

Nach dem Trocken wurde das oberflächennachvernetzte Grundpolymer in einem NARA Paddle-Cooler (GMF Gouda, Waddinxveen, Niederlande) auf ca. 60°C abgekühlt.

Die erhaltenen wasserabsorbierenden Polymerpartikel wiesen eine Zentrifugenretentionskapazität (CRC) von 28,4 g/g auf.

### Beispiel 2

Es wurde verfahren wie unter Beispiel 1. Das Polymergel wurde in der Kühlzone des Umluftbandtrockners auf 80° statt auf 100°C abgekühlt. Die Gastemperatur am Ende der pneumatischen Förderung 1 betrug 60°C statt 75°C.

Die Klassierung lief mehrere Wochen störungsfrei.

### Beispiel 3 (Vergleichsbeispiel)

Es wurde verfahren wie unter Beispiel 1. Das Polymergel wurde in der Kühlzone des Umluftbandtrockners auf 60° statt auf 100°C abgekühlt. Die Gastemperatur am Ende der pneumatischen Förderung 1 betrug 40°C statt 75°C.

Innerhalb weniger Stunden wurden in der Partikelgrößenfraktion von 150 bis 850 µm größere Agglomerate und in der Taumelsiebmaschine Anbackungen an den Wänden beobachtet.

### Beispiel 4 (Vergleichsbeispiel)

Es wurde verfahren wie unter Beispiel 1. Das Polymergel wurde in der Kühlzone des Umluftbandtrockners auf 140° statt auf 100°C abgekühlt. Die Gastemperatur am Ende der pneumatischen Förderung 1 betrug 110°C statt 75°C.

Innerhalb weniger Tage mussten aufgrund von Beschädigungen einzelne Siebe der Taumelsiebmaschine ausgetauscht werden.

## Patentansprüche

1. Verfahren zur Herstellung von Superabsorbern durch Polymerisation einer Monomerlösung oder-suspension, enthaltend
a) ein ethylenisch ungesättigtes, säuregruppentragendes Monomer, das zumindest teilweise neutralisiert ist,
b) mindestens einen Vernetzer,
c) mindestens einen Initiator,
d) optional ein mit den unter a) genannten Monomeren copolymerisierbares ethylenisch ungesättigtes Monomer und
e) optional ein oder mehrere wasserlösliche Polymere,
umfassend die Schritte
i) Polymerisation der Monomerlösung oder -suspension,
ii) optional Zerkleinerung des in Schritt i) erhaltenen Polymergels,
iii) Trocknung des in Schritt i) oder Schritt ii) erhaltenen Polymergels in einem Umluftbandtrockner mit mehreren Heizzonen und mindestens einer Kühlzone,
iv) Brechen des in Schritt iii) erhaltenen getrockneten Polymergels,
v) optional Grobzerkleinung der in Schritt iv) erhaltenen Polymerpartikel,
vi) pneumatischer Förderung der in Schritt iv) oder Schritt v) erhaltenen Polymerpartikel,
vii) optional Abtrennung unvollständig getrockneter Polymerpartikel aus den in Schritt vi) erhaltenen Polymerpartikeln, wobei die verbleibenden getrockneten Polymerpartikel in Schritt viii), Schritt ix) oder Schritt x) weiterverarbeitet werden,
viii) optional Klassierung der in Schritt vi) oder Schritt vii) erhaltenen Polymerpartikel, wobei die Grobfraktion dem Schritt ix) oder Schritt x) zugeführt wird,
ix) optional Zwischenlagerung der in Schritt vi), Schritt vii) oder Schritt viii) erhaltenen Polymerpartikel,
x) Zerkleinerung der Polymerpartikel der in Schritt vi), Schritt vii), Schritt viii) oder Schritt ix) erhaltenen Polymerpartikel,
xi) optional pneumatischer Förderung der in Schritt x) erhaltenen Polymerpartikel,
xii) Klassierung der in Schritt x) oder Schritt xi) erhaltenen Polymerpartikel und
xiii) optional Oberflächennachvernetzung der in Schritt viii) und/oder Schritt xii) erhaltenen klassierten Polymerpartikel,
**dadurch gekennzeichnet, dass** die Gastemperatur am Ende der pneumatischen Förderung in Schritt vi) von 50 bis 95°C beträgt.

2. Verfahren gemäß Anspruch 1, **dadurch gekennzeichnet, dass** die in Schritt vi) erhaltenen Polymerpartikel oder die in oder Schritt vii) nach der Abtrennung der unvollständig getrockneter Polymerpartikel verbleibenden Polymerpartikel in Schritt viii) klassiert werden, wobei die Grobfraktion dem Schritt ix) oder Schritt x) zugeführt wird.

3. Verfahren gemäß Anspruch 1 oder 2, **dadurch gekennzeichnet, dass** die Gastemperatur am Ende der pneumatischen Förderung in Schritt vi) von 59 bis 80°C beträgt.

4. Verfahren gemäß einem der Ansprüche 1 bis 3, **dadurch gekennzeichnet, dass** die Verweilzeit der Polymerpartikel zwischen dem Ende der Trocknung in Schritt iii) und dem Ende der pneumatischen Förderung in Schritt vi) weniger als 30 Minuten beträgt.

5. Verfahren gemäß einem der Ansprüche 1 bis 4, **dadurch gekennzeichnet, dass** der Feuchtegehalt der Polymerpartikel in Schritt x) von 1 bis 10 Gew.-% beträgt.

6. Verfahren gemäß einem der Ansprüche 1 bis 5, **dadurch gekennzeichnet, dass** die Kühlleistung der mindestens einen Kühlzone in Schritt iii) zur Einstellung der Gastemperatur am Ende der pneumatischen Förderung in Schritt vi) verwendet wird.

7. Verfahren gemäß einem der Ansprüche 1 bis 6, **dadurch gekennzeichnet, dass** das getrocknete Polymergel in Schritt iv) mittels einer Stachelwalze oder eines Kreuzflügelzerkleinerers gebrochen wird.

8. Verfahren gemäß einem der Ansprüche 1 bis 7, **dadurch gekennzeichnet, dass** die Polymerpartikel in Schritt x) mittels mindestens eines mehrstufigen Walzenstuhls zerkleinert werden.

9. Verfahren gemäß einem der Ansprüche 1 bis 8, **dadurch gekennzeichnet, dass** die Polymerpartikel in Schritt viii) und/oder Schritt xii) mittels mindestens einer Taumelsiebmaschine klassiert werden.

10. Verfahren gemäß einem der Ansprüche 1 bis 9, **dadurch gekennzeichnet, dass** die in Schritt iv) erhaltenen Polymerpartikel in Schritt v) mittels mindestens eines Walzenbrechers grobzerkleinert werden.

11. Verfahren gemäß einem der Ansprüche 1 bis 10, **dadurch gekennzeichnet, dass** die in Schritt x) erhaltenen Polymerpartikel in Schritt xi) pneumatisch gefördert werden.

12. Verfahren gemäß einem der Ansprüche 1 bis 11, **dadurch gekennzeichnet, dass** aus den in Schritt vi) erhaltenen Polymerpartikel in Schritt vii) unvollständig getrockneter Polymerpartikel abgetrennt werden.

13. Verfahren gemäß einem der Ansprüche 1 bis 12, **dadurch gekennzeichnet, dass** die in Schritt vii) erhaltenen Polymerpartikel in Schritt viii) klassiert werden.

14. Verfahren gemäß einem der Ansprüche 1 bis 13, **dadurch gekennzeichnet, dass** die in Schritt viii) und/oder Schritt xii) erhaltenen Polymerpartikel in Schritt xiii) oberflächennachvernetzt werden.

15. Verfahren gemäß einem der Ansprüche 1 bis 14, **dadurch gekennzeichnet, dass** die Polymerpartikel eine Zentrifugenretentionskapazität von mindestens 15 g/g aufweisen.

## Claims

1. A process for producing superabsorbents by polymerizing a monomer solution or suspension comprising
a) an ethylenically unsaturated monomer which bears acid groups and is at least partly neutralized,
b) at least one crosslinker,
c) at least one initiator,
d) optionally an ethylenically unsaturated monomer copolymerizable with the monomers mentioned under a) and
e) optionally one or more water-soluble polymers, comprising the steps of
i) polymerizing the monomer solution or suspension,
ii) optionally comminuting the polymer gel obtained in step i),
iii) drying the polymer gel obtained in step i) or step ii) in an air circulation belt drier with multiple heating zones and at least one cooling zone,
iv) crushing the dried polymer gel obtained in step iii),
v) optionally coarsely comminuting the polymer particles obtained in step iv),
vi) pneumatically conveying the polymer particles obtained in step iv) or step v),
vii) optionally removing incompletely dried polymer particles from the polymer particles obtained in step vi), and further processing the remaining dried polymer particles in step viii), step ix) or step x),
viii) optionally classifying the polymer particles obtained in step vi) or step vii), and sending the coarse fraction to step ix) or step x),
ix) optionally intermediately storing the polymer particles obtained in step vi), step vii) or step viii),
x) comminuting the polymer particles of the polymer particles obtained in step vi), step vii), step viii) or step ix),
xi) optionally pneumatically conveying the polymer particles obtained in step x),
xii) classifying the polymer particles obtained in step x) or step xi) and
xiii) optionally surface postcrosslinking the classified polymer particles obtained in step viii) and/or step xii),
wherein the gas temperature at the end of the pneumatic conveying in step vi) is from 50 to 95°C.

2. The process according to claim 1, wherein the polymer particles obtained in step vi) or the polymer particles remaining in step vii) after the removal of the incompletely dried polymer particles are classified in step viii), and the coarse fraction is sent to step ix) or step x).

3. The process according to claim 1 or 2, wherein the gas temperature at the end of the pneumatic conveying in step vi) is from 59 to 80°C.

4. The process according to any of claims 1 to 3, wherein the dwell time of the polymer particles between the end of the drying in step iii) and the end of the pneumatic conveying in step vi) is less than 30 minutes.

5. The process according to any of claims 1 to 4, wherein the moisture content of the polymer particles in step x) is from 1% to 10% by weight.

6. The process according to any of claims 1 to 5, wherein the cooling output of the at least one cooling zone in step iii) is used to adjust the gas temperature at the end of the pneumatic conveying in step vi).

7. The process according to any of claims 1 to 6, wherein the dried polymer gel is crushed in step iv) by means of a spiked roller or a cross-blade comminutor.

8. The process according to any of claims 1 to 7, wherein the polymer particles are comminuted in step x) by means of at least one multistage roll mill.

9. The process according to any of claims 1 to 8, wherein the polymer particles are classified in step viii) and/or step xii) by means of at least one tumbler sieving machine.

10. The process according to any of claims 1 to 9, wherein the polymer particles obtained in step iv) are coarsely comminuted in step v) by means of at least one roll crusher.

11. The process according to any of claims 1 to 10, wherein the polymer particles obtained in step x) are conveyed pneumatically in step xi).

12. The process according to any of claims 1 to 11, wherein incompletely dried polymer particles are separated in step vii) from the polymer particles obtained in step vi).

13. The process according to any of claims 1 to 12, wherein the polymer particles obtained in step vii) are classified in step viii).

14. The process according to any of claims 1 to 13, wherein the polymer particles obtained in step viii) and/or step xii) are surface postcrosslinked in step xiii) .

15. The process according to any of claims 1 to 14, wherein the polymer particles have a centrifuge retention capacity of at least 15 g/g.

## Revendications

1. Procédé de fabrication de superabsorbants par polymérisation d'une solution ou suspension de monomères, contenant
a) un monomère éthyléniquement insaturé, portant des groupes acide, qui est au moins partiellement neutralisé,
b) au moins un agent de réticulation,
c) au moins un initiateur,
d) éventuellement un monomère éthyléniquement insaturé copolymérisable avec les monomères indiqués en a), et
e) éventuellement un ou plusieurs polymères solubles dans l'eau,
comprenant les étapes suivantes :
i) la polymérisation de la solution ou suspension de monomères,
ii) éventuellement le broyage du gel polymère obtenu dans l'étape i),
iii) le séchage du gel polymère obtenu dans l'étape i) ou dans l'étape ii) dans un séchoir à bande à circulation d'air muni de plusieurs zones de chauffage et d'au moins une zone de refroidissement,
iv) le concassage du gel polymère séché obtenu dans l'étape iii),
v) éventuellement le broyage grossier des particules polymères obtenues dans l'étape iv),
vi) le transport pneumatique des particules polymères obtenues dans l'étape iv) ou dans l'étape v),
vii) éventuellement la séparation de particules polymères incomplètement séchées des particules polymères obtenues dans l'étape vi), les particules polymères séchées restantes étant traitées ultérieurement dans l'étape viii), dans l'étape ix) ou dans l'étape x),
viii) éventuellement la classification des particules polymères obtenues dans l'étape vi) ou dans l'étape vii), la fraction grossière étant acheminée dans l'étape ix) ou dans l'étape x),
ix) éventuellement l'entreposage des particules polymères obtenues dans l'étape vi), dans l'étape vii) ou dans l'étape viii),
x) le broyage des particules polymères des particules polymères obtenues dans l'étape vi), dans l'étape vii), dans l'étape viii) ou dans l'étape ix),
xi) éventuellement le transport pneumatique des particules polymères obtenues dans l'étape x),
xii) la classification des particules polymères obtenues dans l'étape x) ou dans l'étape xi), et
xiii) éventuellement la post-réticulation de surface des particules polymères classifiées obtenues dans l'étape viii) ou dans l'étape xii),
**caractérisé en ce que** la température du gaz à la fin du transport pneumatique dans l'étape vi) est de 50 à 95 °C.

2. Procédé selon la revendication 1, **caractérisé en ce que** les particules polymères obtenues dans l'étape vi) ou les particules polymères restantes dans l'étape vii) après la séparation des particules polymères incomplètement séchées sont classifiées dans l'étape viii), la fraction grossière étant acheminée dans l'étape ix) ou dans l'étape x).

3. Procédé selon la revendication 1 ou 2, **caractérisé en ce que** la température du gaz à la fin du transport pneumatique dans l'étape vi) est de 59 à 80 °C.

4. Procédé selon l'une quelconque des revendications 1 à 3, **caractérisé en ce que** le temps de séjour des particules polymères entre la fin du séchage dans l'étape iii) et la fin du transport pneumatique dans l'étape vi) est de moins de 30 minutes.

5. Procédé selon l'une quelconque des revendications 1 à 4, **caractérisé en ce que** la teneur en humidité des particules polymères dans l'étape x) est de 1 à 10 % en poids.

6. Procédé selon l'une quelconque des revendications 1 à 5, **caractérisé en ce que** la puissance de refroidissement de ladite au moins une zone de refroidissement dans l'étape iii) est utilisée pour ajuster la température du gaz à la fin du transport pneumatique dans l'étape vi).

7. Procédé selon l'une quelconque des revendications 1 à 6, **caractérisé en ce que** le gel polymère séché est concassé dans l'étape iv) au moyen d'un rouleau à pointes ou d'un broyeur à ailettes croisées.

8. Procédé selon l'une quelconque des revendications 1 à 7, **caractérisé en ce que** les particules polymères sont broyées dans l'étape x) au moyen d'au moins un moulin à cylindres à plusieurs niveaux.

9. Procédé selon l'une quelconque des revendications 1 à 8, **caractérisé en ce que** les particules polymères sont classifiées dans l'étape viii) et/ou dans l'étape xii) au moyen d'au moins un tamiseur à nutation.

10. Procédé selon l'une quelconque des revendications 1 à 9, **caractérisé en ce que** les particules polymères obtenues dans l'étape iv) sont broyées grossièrement dans l'étape v) au moyen d'au moins un concasseur à cylindres.

11. Procédé selon l'une quelconque des revendications 1 à 10, **caractérisé en ce que** les particules polymères obtenues dans l'étape x) sont transportées pneumatiquement dans l'étape xi).

12. Procédé selon l'une quelconque des revendications 1 à 11, **caractérisé en ce que** des particules polymères incomplètement séchées sont séparées dans l'étape vii) des particules polymères obtenues dans l'étape vi).

13. Procédé selon l'une quelconque des revendications 1 à 12, **caractérisé en ce que** les particules polymères obtenues dans l'étape vii) sont classifiées dans l'étape viii).

14. Procédé selon l'une quelconque des revendications 1 à 13, **caractérisé en ce que** les particules polymères obtenues dans l'étape viii) et/ou dans l'étape xii) sont post-réticulées en surface dans l'étape xiii).

15. Procédé selon l'une quelconque des revendications 1 à 14, **caractérisé en ce que** les particules polymères présentent une capacité de rétention centrifuge d'au moins 15 g/g.
